(19) Europäisches Patentamt
European Patent Office
Office européen des brevets

(11) **EP 3 801 668 B1**

(12) **EUROPÄISCHE PATENTSCHRIFT**

(45) Veröffentlichungstag und Bekanntmachung des Hinweises auf die Patenterteilung:
**07.02.2024 Patentblatt 2024/06**

(21) Anmeldenummer: **19729729.4**

(22) Anmeldetag: **06.06.2019**

(51) Internationale Patentklassifikation (IPC):
*A61B 8/06* (2006.01)    *A61B 8/12* (2006.01)
*A61B 8/08* (2006.01)    *A61B 5/00* (2006.01)
*A61B 5/026* (2006.01)    *A61M 60/237* (2021.01)
*A61M 60/13* (2021.01)    *A61M 60/523* (2021.01)
*A61M 60/816* (2021.01)    *A61M 60/857* (2021.01)
*A61M 60/178* (2021.01)    *A61M 60/226* (2021.01)
*A61B 5/029* (2006.01)

(52) Gemeinsame Patentklassifikation (CPC):
**A61B 5/686; A61B 5/029; A61B 8/06; A61B 8/12;
A61B 8/488; A61M 60/13; A61M 60/178;
A61M 60/226; A61M 60/237; A61M 60/523;
A61M 60/816; A61M 60/857;** A61B 8/0883;
A61B 8/0891; A61M 2205/3334;        (Forts.)

(86) Internationale Anmeldenummer:
**PCT/EP2019/064807**

(87) Internationale Veröffentlichungsnummer:
**WO 2019/234166 (12.12.2019 Gazette 2019/50)**

(54) **VERFAHREN ZUR BESTIMMUNG EINER STRÖMUNGSGESCHWINDIGKEIT EINES DURCH EIN IMPLANTIERTES, VASKULÄRES UNTERSTÜTZUNGSSYSTEM STRÖMENDEN FLUIDS UND IMPLANTIERBARES, VASKULÄRES UNTERSTÜTZUNGSSYSTEM**

METHOD FOR DETERMINING A FLOW SPEED OF A FLUID FLOWING THROUGH AN IMPLANTED, VASCULAR ASSISTANCE SYSTEM AND IMPLANTABLE, VASCULAR ASSISTANCE SYSTEM

PROCÉDÉ DE DÉTERMINATION DE LA VITESSE D'ÉCOULEMENT D'UN FLUIDE S'ÉCOULANT À TRAVERS UN SYSTÈME D'ASSISTANCE VASCULAIRE IMPLANTÉ ET SYSTÈME D'ASSISTANCE VASCULAIRE IMPLANTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018 DE 102018208933**

(43) Veröffentlichungstag der Anmeldung:
**14.04.2021 Patentblatt 2021/15**

(73) Patentinhaber: **Kardion GmbH**
**70376 Stuttgart (DE)**

(72) Erfinder:
• **SCHLEBUSCH, Thomas Alexander**
**71272 Renningen (DE)**

• **SCHMID, Tobias**
**70197 Stuttgart (DE)**

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
**DE-A1- 10 060 275      DE-A1-102015 004 177
US-A1- 2014 100 414      US-A1- 2015 141 832**

(52) Gemeinsame Patentklassifikation (CPC): (Forts.)
A61M 2205/3375; A61M 2230/04

**Beschreibung**

Die Erfindung betrifft ein Verfahren zur Bestimmung einer Strömungsgeschwindigkeit eines durch ein implantiertes, vaskuläres Unterstützungssystem strömenden Fluids, ein implantierbares, vaskuläres Unterstützungssystem sowie eine Verwendung eines Betriebsparameters einer Strömungsmaschine eines implantierten, vaskulären Unterstützungssystems. Die Erfindung findet insbesondere Anwendung bei (voll-)implantierten Linksherz-Unterstützungssystemen (LVAD).

Die DE 100 60 275 A1 beschreibt ein implantierbares vaskuläres Unterstützungssystem mit einem Pumpengehäuse und mit einem Ultraschall-Dopplersensor, der für das Bestimmen der Blutströmung in dem Pumpengehäuse dient.

Es ist bekannt Ultraschall-Volumenstromsensoren in Herzunterstützungssystemen zu integrieren, um mit ihnen den sog. Pumpenvolumenstrom zu erfassen, der den Fluid-Volumenstrom durch das Unterstützungssystem selbst quantifiziert. Die Ultraschall-Volumenstromsensoren können dabei gepulste Dopplermessungen durchführen bzw. das gepulste Doppler-(engl.: Pulsed Wave Doppler; kurz: PWD)-Verfahren einsetzen. Dieses benötigt nur ein Ultraschall-Wandlerelement und ermöglicht eine genaue Wahl des Abstandes des Beobachtungsfensters vom Ultraschall-Element.

Die Aufgabe eines kardialen Unterstützungssystems ist die Förderung von Blut. Hierbei hat das sog. Herz-Zeit-Volumen (HZV, üblicherweise angegeben in Liter pro Minute) eine hohe klinische Relevanz. Das Herz-Zeit-Volumen betrifft hierbei mit anderen Worten den Gesamtvolumenstrom an Blut aus einem Ventrikel, insbesondere vom linken Ventrikel hin zur Aorta. Entsprechend eingängig ist das Bestreben, diesen Parameter als Messwert während des Betriebs eines kardialen Unterstützungssystems zu erheben.

Je nach Unterstützungsgrad, der den Anteil des durch ein Fördermittel wie etwa eine Pumpe des Unterstützungssystems geförderten Volumenstroms zum Gesamtvolumenstrom an Blut vom Ventrikel hin zur Aorta beschreibt, gelangt ein gewisser Volumenstrom über den physiologischen Weg durch die Aortenklappe in die Aorta. Das Herz-Zeit-Volumen bzw. der Gesamtvolumenstrom ($Q_{HZV}$) vom Ventrikel hin zur Aorta ist demnach üblicherweise die Summe aus Pumpenvolumenstrom ($Q_p$) und Aortenklappen-Volumenstrom ($Q_a$).

Ein etabliertes Verfahren zur Bestimmung des Herz-Zeit-Volumens ($Q_{HZV}$) im klinischen Umfeld ist die Verwendung von Dilutionsverfahren, die jedoch alle auf einen transkutan eingeführten Katheter setzen und daher nur während einer Herzoperation und im anschließenden intensivmedizinischen Aufenthalt Herz-Zeit-Volumen-Messdaten liefern können. Für hohe Unterstützungsgrade geht Qa gegen Null, sodass annähernd $Q_p \approx Q_{HZV}$ gilt. Demnach kann zumindest in diesen Fällen das Herz-Zeit-Volumen zumindest annäherungsweise über den Pumpenvolumenstrom bestimmt werden. Ein etabliertes Verfahren zur Messung des Pumpenvolumenstroms ($Q_p$) ist die Korrelation aus den Betriebsparametern des Unterstützungssystems, vor allem der elektrischen Leistungsaufnahme, eventuell ergänzt um weitere physiologische Parameter wie den Blutdruck. Da diese Verfahren auf statistischen Annahmen und dem zugrunde liegenden Pumpen-Kennfeld des verwendeten Unterstützungssystems basieren, sind die korrelierten $Q_p$ fehlerbehaftet. Zur Steigerung der Messqualität des Parameters $Q_p$ ist daher die Aufnahme eines Flusssensors wünschenswert.

Ein besonders geeignetes Sensorverfahren zur Bestimmung von Strömungsgeschwindigkeiten und damit auch Volumenströmen ist Ultraschall, im

Besonderen das Pulsed Wave Doppler-Verfahren (PWD), da es nur ein bidirektionales Ultraschall-Wandlerelement benötigt und eine genaue Wahl des Abstandes des Beobachtungsfensters ermöglicht, in dem die Messwerte erhoben werden. Somit ist es möglich, die Flussgeschwindigkeitsmessung in dem Bereich durchzuführen, in dem geeignete Strömungsverhältnisse herrschen.

Bei einem PWD-System werden Ultraschallimpulse mit einer festgelegten Pulswiederholrate (PRF) ausgesendet. Sind Strömungsgeschwindigkeit und Strömungsrichtung unbekannt, muss die PRF mindestens die doppelte, maximal auftretende Doppler-Frequenzverschiebung übertreffen, um das Nyquist-Theorem nicht zu verletzten. Ist diese Bedingung nicht erfüllt, kommt es zu Aliasing, d. h. Doppeldeutigkeiten im bestimmten Frequenzspektrum. Bei der Detektion einer Frequenz im Frequenzspektrum, kann diese nicht mehr eindeutig einer sondern mehreren Flussgeschwindigkeiten zugeordnet werden.

Aufgrund der geometrischen Gestaltung des Messaufbaus in Herzunterstützungssystemen (VAD) liegt der Messbereich bzw. das Beobachtungsfenster unter Umständen derart weit vom Ultraschallwandler entfernt, dass die Signallaufzeit des Ultraschallimpulses vom Wandler zum Messbereich und zurück zum Wandler nicht vernachlässigt werden kann. Da ein neuer Ultraschallimpuls erst ausgesendet werden darf, wenn der vorangegangene keine signifikanten Echos mehr liefert, limitiert die Signallaufzeit die maximal mögliche PRF. Bei den hohen in Herzunterstützungssystemen vorherrschenden Strömungsgeschwindigkeiten und den geometrischen Randbedingungen für die Entfernung des Beobachtungsfensters vom Ultraschallelement, kommt es zwangsläufig zu einer Verletzung des Nyquist-Abtasttheorems wodurch Doppeldeutigkeiten im Spektrum entstehen.

Herzunterstützungssysteme mit Ultraschallsensoren, die nicht das PWD-Verfahren anwenden, sind üblicherweise mit zwei Ultraschallwandlern ausgestattet, sodass die beschriebene Laufzeitproblematik zwar auftreten kann, aber bei entsprechender Umsetzung anderweitig gelöst werden kann. Herzunterstützungssysteme mit Ultraschallsensoren, die das PWD-Verfahren anwenden sind jedoch insbesondere für mittlere bis hohe Flussgeschwindigkeiten anfällig für den beschriebenen Effekt. Stand der Technik ist derzeit die Maßgabe, die festgelegte Pulswiederholrate so zu wählen, dass Aliasing nicht auftritt bzw. sowohl die geometrischen Bedingungen und die Ultraschallfrequenz falls möglich geeignet einzustellen.

Aufgabe der Erfindung ist es, ein verbessertes Verfahren zur Bestimmung einer Strömungsgeschwindigkeit eines durch ein implantiertes, vaskuläres Unterstützungssystem strömenden Fluids anzugeben und ein verbessertes implantierbares vaskuläres Unterstützungssystem zu schaffen, in dem die einer Strömungsgeschwindigkeit eines durch dieses strömenden Fluids bestimmt werden kann.

Insbesondere ist es eine Aufgabe der Erfindung, ein Verfahren zur Bestimmung einer Strömungsgeschwindigkeit eines Fluids und ein verbessertes implantierbares vaskuläres Unterstützungssystem zu schaffen, in dem das Bestimmen der Strömungsgeschwindigkeit eines durch dieses strömenden Fluids vorgesehen ist, bei dem mit nur einem Ultraschallwandler das Bestimmen der Strömungsgeschwindigkeit bei den in einem Herzunterstützungssystem herrschenden Strömungsgeschwindigkeiten auch bei großer Signallaufzeit eines Ultraschallimpulses von dem Ultraschallwandler zum Messbereich und zurück möglich ist.

Diese Aufgabe wird durch das in Anspruch 1 angegebene Verfahren und das implantierbare, vaskuläre Unterstützungssystem nach Anspruch 7 gelöst.

Vorteilhafte Ausführungsformen der Erfindung sind in den abhängigen Ansprüchen angegeben.

Hier vorgeschlagen wird gemäß Anspruch 1 ein Verfahren zur Bestimmung zumindest einer Strömungsgeschwindigkeit oder eines Fluid-Volumenstroms eines durch ein implantiertes, vaskuläres Unterstützungssystem strömenden Fluids, umfassend folgende Schritte:

a) Durchführen einer gepulsten Dopplermessung mittels eines Ultraschallsensors des Unterstützungssystems,
b) Auswerten eines Messergebnisses aus Schritt a), welches eine mögliche Mehrdeutigkeit aufweist,
c) Bereitstellen mindestens eines Betriebsparameters einer Strömungsmaschine des Unterstützungssystems,
d) Ermitteln zumindest der Strömungsgeschwindigkeit oder des Fluid-Volumenstroms unter Verwendung des in Schritt b) ausgewerteten Messergebnisses,

wobei die mögliche Mehrdeutigkeit des Messergebnisses unter Verwendung des Betriebsparameters korrigiert wird.

Das vaskuläre Unterstützungssystem ist bevorzugt ein kardiales Unterstützungssystem, besonders bevorzugt ein ventrikuläres Unterstützungssystem. Regelmäßig dient das Unterstützungssystem zur Unterstützung der Förderung von Blut im Blutkreislauf eines Menschen, ggf. Patienten. Das Unterstützungssystem kann zumindest teilweise in einem Blutgefäß angeordnet sein. Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet.

Die hier vorgeschlagene Lösung trägt insbesondere zur Bereitstellung eines Aliasing-Kompensationsverfahrens für einen Ultraschall-Volumenstromsensor in einem Herzunterstützungssystem bei. Das Verfahren kann zur Bestimmung einer Fluid-Strömungsgeschwindigkeit und/oder eines Fluid-Volumenstroms aus einem Ventrikel eines Herzens, insbesondere von einem (linken) Ventrikel eines Herzens hin zur Aorta im Bereich eines (voll-)implantierten, (links-)ventrikulären (Herz-)Unterstützungssystems beitragen. Bei dem Fluid handelt es sich regelmäßig um Blut. Die Strömungsgeschwindigkeit wird in einem Fluidstrom bzw. Fluid-Volumenstrom bestimmt, der durch das Unterstützungssystem, insbesondere durch ein (Zulauf-)Rohr bzw. eine (Zulauf-)Kanüle des Unterstützungssystems hindurch strömt. Das Verfahren ermöglicht in vorteilhafter Weise, dass die Strömungsgeschwindigkeit und/oder der Fluid-Volumenstrom des Blut-Flusses auch außerhalb des OP-Szenarios mit hoher Qualität bestimmt werden kann, insbesondere durch das implantierte Unterstützungssystem selbst.

Bei der hier vorgeschlagenen Lösung kann in besonders vorteilhafter Weise der Sachverhalt genutzt werden, dass aufgrund des Motorkennfeldes eine grobe Abschätzung des Pumpenflusses (nur) aus der Drehrate des Antriebs oder auf Basis des Differenzdrucks über die Strömungsmaschine und der Drehrate möglich ist. Die insbesondere grobe Abschätzung der Flussrate aus den Betriebsparametern der Strömungsmaschine wird insbesondere dazu genutzt, die Mehrdeutigkeiten im Spektrum aufzulösen und eine hochgenaue Flussmessung durch den Ultraschallsensor zu ermöglichen.

In Schritt a) erfolgt ein Durchführen einer gepulsten Dopplermessung mittels eines Ultraschallsensors des Unterstützungssystems. Zur Durchführung der gepulsten Dopplermessung wird insbesondere das gepulste Doppler-(engl.: Pulsed Wave Doppler; kurz: PWD)-Verfahren eingesetzt. Insbesondere wird in Schritt a) ein PWD-Messzyklus durchlaufen.

In Schritt b) erfolgt ein Auswerten eines Messergebnisses aus Schritt a), welches eine mögliche Mehrdeutigkeit aufweist. "Mögliche Mehrdeutigkeit" bedeutet mit anderen Worten insbesondere, dass das Messergebnis bzw. alle Messergebnisse nicht zwingend immer eine Mehrdeutigkeit aufweisen müssen. Insbesondere bei einer vergleichsweise hohen Strömungsgeschwindigkeit, wie sie in den hier in Rede stehenden Unterstützungssystemen üblicherweise vorkommt, weist das Messergebnis in der Regel eine Mehrdeutigkeit auf. Bei einer vergleichsweise geringen Strömungsgeschwindigkeit kann es jedoch auch vorkommen, dass das Messergebnis eindeutig ist.

Weiterhin kann das Messergebnis insbesondere nach Schritt b) bereitgestellt werden. Hierbei kann das Messergebnis beispielsweise als Rohdaten (z. B. Frequenzspektrum) bzw. als Rohmessergebnis oder als bereits zumindest teilweise vorverarbeitetes Messergebnis (z. B. als eine (gemessene) Strömungsgeschwindigkeit und/oder als einen (gemessener) Fluid-Volumenstrom) bereitgestellt werden. Das Messergebnis kann beispielsweise einer Verarbeitungseinheit des Unterstützungssystems bereitgestellt werden.

In Schritt c) erfolgt ein Bereitstellen mindestens eines Betriebsparameters einer Strömungsmaschine des Unterstützungssystems. Der Betriebsparameter kann beispielsweise einer Verarbeitungseinheit des Unterstützungssystems bereitgestellt werden. Das in Schritt b) bereitgestellte Messergebnis und der in Schritt c) bereitgestellte Betriebsparameter werden grundsätzlich bezüglich derselben Fluidströmung, etwa in demselben (zeitlichen und/oder räumlichen) Beobachtungsfenster erfasst. Dies bedeutet mit anderen Worten insbesondere, dass das in Schritt b) bereitgestellte Messergebnis und der in Schritt c) bereitgestellte Betriebsparameter sich auf im Wesentlichen denselben Messzeitpunkt beziehen bzw. im Wesentlichen denselben Zeitstempel aufweisen und/oder sich auf denselben Messort beziehen. "Im Wesentlichen" beschreibt hierbei insbesondere eine Abweichung von weniger als einer Sekunde. Dabei kann eine (in der Regel weniger als eine Sekunde betragende) Zeitdifferenz berücksichtigt werden, bis sich der Betriebsparameter (bzw. eine Änderung dessen) auf den Messort auswirkt. Dies kann auch derart beschrieben werden, dass das in Schritt b) bereitgestellte Messergebnis und der in Schritt c) bereitgestellte Betriebsparameter zueinander zugehörig sind. Bevorzugt wird in Schritt c) mindestens ein zu dem in Schritt b) bereitgestellten Messergebnis zugehöriger Betriebsparameter bereitgestellt.

In Schritt d) erfolgt ein Ermitteln der (tatsächlichen) Strömungsgeschwindigkeit unter Verwendung des in Schritt b) ausgewerteten Messergebnisses. Wenn in Schritt b) ein Rohmessergebnis ausgewertet und anschließend bereitgestellt wird, ist es besonders vorteilhaft, wenn hieraus (z. B. in Schritt d) eine (gemessene) Strömungsgeschwindigkeit bestimmt wird. Wenn in Schritt b) ein zu einer (gemessenen) Strömungsgeschwindigkeit vorverarbeitetes Messergebnis bereitgestellt wird, kann dies vorteilhaft unmittelbar in Schritt d) verwendet werden. Die (gemessene) Strömungsgeschwindigkeit ist in der Regel nicht eindeutig. Ferner ist es vorteilhaft, wenn auf Basis des in Schritt c) bereitgestellten Betriebsparameters eine geschätzte Strömungsgeschwindigkeit bestimmt wird. Die (tatsächliche) Strömungsgeschwindigkeit kann nun beispielsweise dadurch ermittelt werden, dass diejenige gemessene Strömungsgeschwindigkeit ausgewählt wird, die am Nächsten an der geschätzten Strömungsgeschwindigkeit liegt.

Alternativ oder kumulativ kann in Schritt d) (statt der Strömungsgeschwindigkeit) ein (tatsächlicher) Fluid-Volumenstrom ermittelt werden. Wenn in Schritt b) ein Rohmessergebnis bereitgestellt wird, ist es dabei besonders vorteilhaft, wenn hieraus ein (gemessener) Fluid-Volumenstrom bestimmt wird. Wenn in Schritt b) ein zu einem (gemessenen) Fluid-Volumenstrom vorverarbeitetes Messergebnis bereitgestellt wird, kann dies vorteilhaft unmittelbar in Schritt d) verwendet werden. Der (gemessene) Fluid-Volumenstrom ist in der Regel nicht eindeutig. Ferner ist es vorteilhaft, wenn auf Basis des in Schritt c) bereitgestellten Betriebsparameters ein geschätzter Fluid-Volumenstrom bestimmt wird. Der (tatsächliche) Fluid-Volumenstrom kann nun beispielsweise dadurch ermittelt werden, dass derjenige gemessene Fluid-Volumenstrom ausgewählt wird, der am Nächsten an dem geschätzten Fluid-Volumenstrom liegt.

Im Sinne der hier vorgeschlagenen Lösung wird die mögliche Mehrdeutigkeit des Messergebnisses unter Verwendung des Betriebsparameters korrigiert bzw. aufgelöst. Das Messergebnis ist in der Regel mehrdeutig. Diese Mehrdeutigkeit lässt sich insbesondere mit der hier in der Regel vorliegenden Verletzung des Nyquist-Abtasttheorems erklären. Diese Verletzung des Nyquist-Abtasttheorems wird insbesondere dadurch verursacht, dass in dem Unterstützungssystem zwischen Ultraschallsensor und Beobachtungsfenster bzw. Messbereich vergleichsweise lange Signallaufzeiten bestehen und bei den gepulsten Dopplermessungen in der Regel ein neuer Ultraschall-Impuls erst ausgesendet wird, wenn das Echo eines unmittelbar zuvor ausgesendeten Ultraschall-Impulses empfangen wurde bzw. abgeklungen ist.

Die Korrektur bzw. das Auflösen der möglichen Mehrdeutigkeit kann beispielsweise in Schritt d) erfolgen. In diesem Zusammenhang kann in Schritt d) ein Ermitteln der Strömungsgeschwindigkeit unter Verwendung des in Schritt b) ausgewerteten und/oder bereitgestellten (ggf. mehrdeutigen) Messergebnisses und des in Schritt c) bereitgestellten Betriebsparameters erfolgen, wobei die mögliche Mehrdeutigkeit des Messergebnisses unter Verwendung des Betriebsparameters korrigiert wird. Eine Möglichkeit, um eine solche Korrektur durchzuführen bzw. eine mögliche Mehrdeutigkeit aufzulösen wurde vorstehend bereits beschrieben. Hierbei wird beispielhaft diejenige gemessene Strömungsgeschwindigkeit bzw. derjenige gemessene Fluid-Volumenstrom ausgewählt wird, der am Nächsten an der geschätzten Strömungsgeschwindigkeit bzw. dem geschätzten Fluid-Volumenstrom liegt.

Alternativ kann die Korrektur bzw. das Auflösen der möglichen Mehrdeutigkeit beispielweise (bereits) in Schritt b) erfolgen. Diese Alternative kann auch als a priori Abschätzung oder als a priori Selektion bzw. Vorselektion bezeichnet werden. Dies bedeutet mit anderen Worten insbesondere, dass die Korrektur bzw. das Auflösen der möglichen Mehrdeutigkeit bereits während dem Auswerten des Messergebnisses erfolgt. Dies kann besonders vorteilhaft derart geschehen, dass (nur) der Bereich bzw. Abschnitt des (Roh-)Messergebnisses ausgewertet wird, in dem ein plausibles Ergebnis zu erwarten ist. In Schritt b) kann in diesem Fall das ausgewertete (nicht mehr mehrdeutige) Messergebnis bereitgestellt werden. In Schritt d) kann in diesem Fall das ausgewertete (nicht mehr mehrdeutige) Messergebnis verwendet werden.

"A priori" bedeutet hier insbesondere, dass der Betriebsparameter bereitgestellt und/oder die geschätzte Strömungsgeschwindigkeit bzw. der geschätzte Fluid-Volumenstrom ermittelt wird, bevor das (möglicherweise mehrdeutige) Messergebnis ausgewertet (und ggf. bereitgestellt) wird. Beispielsweise kann der Betriebsparameter, die a priori geschätzte Strömungsgeschwindigkeit und/oder der a priori geschätzte Fluid-Volumenstrom (ggf. in der Art einer Fensterfunktion bzw. Fensterung) zu einer Vorselektion beitragen, um nur ein plausibles Messergebnis oder nur den plausiblen Teil des Messergebnisses auszuwerten und/oder bereitzustellen. Hierzu kann beispielsweise ein (reflektierter und dann) empfangener Ultraschallimpuls nur in dem (Frequenz-)Abschnitt ausgewertet werden, in dem ein plausibles Ergebnis zu erwarten ist.

Nach einer vorteilhaften Ausgestaltung wird vorgeschlagen, dass in Schritt a) ein neuer Ultraschall-Impuls erst ausgesendet wird, wenn ein Echo eines unmittelbar zuvor ausgesendeten Ultraschall-Impulses (ausreichend) abgeklungen ist und/oder empfangen wurde. Bevorzugt wird ein neuer Ultraschall-Impuls erst ausgesendet, wenn alle (signifikanten) Echos eines unmittelbar zuvor ausgesendeten Ultraschall-Impulses (ausreichend) abgeklungen sind und/oder empfangen wurden. Weiterhin bevorzugt wird ein neuer Ultraschall-Impuls erst ausgesendet, wenn die (signifikanten) Echos eines unmittelbar zuvor ausgesendeten Ultraschall-Impulses aus einem (vordefinierten) Messfenster bzw. Messbereich (ausreichend) abgeklungen sind und/oder empfangen wurden.

Eine maximale Pulswiederholrate der gepulsten Dopplermessung ist kleiner als das Zweifache einer maximal auftretenden Doppler-Verschiebung. Vorzugsweise ist die maximale Pulswiederholrate der gepulsten Dopplermessungen kleiner als die maximal auftretende bzw. zu erwartende Doppler-Verschiebung. Wenn die maximale Pulswiederholrate kleiner ist als das Zweifache der maximal auftretenden Doppler-Verschiebung erfolgt grundsätzlich eine Verletzung des Nyquist-Abtasttheorems. Diese Verletzung kann jedoch erforderlich sein, um in einem vaskulären Unterstützungssystem ein PWD-Verfahren ausführen zu können.

Nach einer vorteilhaften Ausgestaltung wird vorgeschlagen, dass der Betriebsparameter zumindest eine Drehzahl, ein Strom, eine Leistung oder ein Druck ist. Bevorzugt ist der Betriebsparameter eine Drehzahl (bzw. Drehrate) der Strömungsmaschine, etwa eines Antriebs (z. B. eines Elektromotors) und/oder eines Schaufelrads der Strömungsmaschine. Weiterhin bevorzugt umfasst der mindestens eine Betriebsparameter eine Drehzahl der Strömungsmaschine und einen Differenzdruck über die Strömungsmaschine.

Nach einer vorteilhaften Ausgestaltung wird vorgeschlagen, dass mit dem Betriebsparameter zumindest eine geschätzte Strömungsgeschwindigkeit oder ein geschätzter Fluid-Volumenstrom (a priori) ermittelt wird. Dies kann beispielsweise unter Verwendung eines Kennfeldes erfolgen, in dem die geschätzte Strömungsgeschwindigkeit bzw. der geschätzte Fluid-Volumenstrom in Abhängigkeit von dem mindestens einen Betriebsparamater hinterlegt ist.

Nach einer vorteilhaften Ausgestaltung wird vorgeschlagen, dass mit dem Betriebsparameter (a priori) ein plausibler Bereich bestimmt wird, in welchem plausible Messergebnisse liegen können. In diesem Zusammenhang kann eine Fensterfunktion bzw. Fensterung bei der Frequenzanalyse (z. B. mittels diskreter Fouriertransformation) des (reflektierten und dann) empfangenen Ultraschallimpulses zum Einsatz kommen. Bevorzugt wird ein sog. Hamming-Fenster verwendet. Die Fensterung, insbesondere das Hamming-Fenster, kann in vorteilhafter Weise in Abhängigkeit des Betriebsparameters und/oder der (auf Basis des Betriebsparameters) zu erwartenden und/oder geschätzten Strömungsgeschwindigkeit und/oder des (auf Basis des Betriebsparameters) zu erwartenden und/oder geschätzten Fluid-Volumenstroms gebildet werden.

Nach einer vorteilhaften Ausgestaltung wird vorgeschlagen, dass unter Verwendung der Strömungsgeschwindigkeit ein Fluid-Volumenstrom durch das Unterstützungssystem ermittelt wird. Dies betrifft mit anderen Worten insbesondere einen Fluid-Volumenstrom der (nur) durch das Unterstützungssystems selbst, beispielsweise durch ein (Zulauf-)Rohr bzw. eine (Zulauf-)Kanüle des Unterstützungssystems hindurch fließt. Bei diesem Fluid-Volumenstrom handelt es sich üblicherweise um den sog. Pumpenvolumenstrom ($Q_p$), der nur den Fluss durch das Unterstützungssystem selbst quantifiziert. Ist dieser Wert zusätzlich zu dem Gesamtvolumenstrom bzw. Herz-Zeit-Volumen ($Q_{HZV}$) bekannt, so kann aus dem Verhältnis von $Q_p$ zu $Q_{HZV}$ (d. h. $Q_p/Q_{HZV}$) der sogenannte Unterstützungsgrad berechnet werden. Zur Bestimmung des Fluid-Volumenstroms kann die ermittelte Strömungsgeschwindigkeit beispielsweise mit einem durchströmbaren Querschnitt des Unterstützungssystems, insbesondere einem durchströmbaren Rohr- bzw. Kanülen-Querschnitt multipliziert werden.

Nach einem weiteren Aspekt wird ein implantierbares, vaskuläres Unterstützungssystem, umfassend:

- einen Ultraschallsensor, eingerichtet zur Durchführung einer gepulsten Dopplermessung,
- eine Strömungsmaschine,
- eine Verarbeitungseinheit, eingerichtet zur Korrektur einer möglichen Mehrdeutigkeit eines Messergebnisses des Ultraschallsensors unter Verwendung des Betriebsparameters der Strömungsmaschine.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere der Ultraschallsensor sich vollständig im Körper des Patienten befinden und dort verbleiben. Das Unterstützungssystem kann auch mehrteilig bzw. mit mehreren, beabstandet voneinander anordenbaren Komponenten ausgeführt sein, sodass beispielsweise der Ultraschallsensor und die Verarbeitungseinheit (Messeinheit) durch ein Kabel separiert voneinander angeordnet sein können. Bei der mehrteiligen Ausführung kann die separat von dem Ultraschallsensor angeordnete Verarbeitungseinheit ebenfalls implantiert oder aber außerhalb des Körpers des Patienten angeordnet werden. Es ist jedenfalls nicht zwingend erforderlich, dass auch die Verarbeitungseinheit im Körper des Patienten angeordnet wird. Beispielsweise kann das Unterstützungssystem so implantiert werden, dass die Verarbeitungseinheit auf der Haut des Patienten bzw. außerhalb des Körpers des Patienten angeordnet wird und eine Verbindung zu dem im Körper angeordneten Ultraschallsensor hergestellt wird. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Weiterhin bevorzugt umfasst das Unterstützungssystem ein Rohr (bzw. eine Kanüle), insbesondere Zulaufrohr bzw. Zulaufkanüle, eine Strömungsmaschine, wie etwa eine Pumpe und/oder einen Elektromotor. Der Elektromotor ist dabei regelmäßig ein Bestandteil der Strömungsmaschine. Das (Zulauf-)Rohr bzw. die (Zulauf-)Kanüle ist vorzugsweise so eingerichtet, dass sie im implantierten Zustand Fluid aus einem (linken) Ventrikel eines Herzens hin zu der Strömungsmaschine führen kann. Das Unterstützungssystem ist vorzugsweise länglich und/oder schlauchartig gebildet. Bevorzugt sind das Rohr (bzw. die Kanüle) und die Strömungsmaschine im Bereich einander gegenüberliegender Enden des Unterstützungssystems angeordnet.

Es ist insbesondere genau bzw. nur ein Ultraschalsensor vorgesehen. Der Ultraschallsensor weist bevorzugt genau bzw. nur ein Ultraschall-Wandlerelement auf. Dies ist insbesondere dann ausreichend für eine Dopplermessung, wenn dabei das PWD-Verfahren zum Einsatz kommt.

Die Strömungsmaschine ist vorzugsweise zumindest in der Art einer Pumpe oder eines (Axial- bzw. Radial-)Verdichters ausgeführt. Die Strömungsmaschine kann zumindest einen ihrer (aktuellen) Betriebsparameter der Verarbeitungseinheit bereitstellen. Darüber hinaus kann eine Steuereinheit zum Steuern bzw. Regeln der Strömungsmaschine vorgesehen sein, die beispielsweise zumindest eine Drehzahl oder eine Leistung der Strömungsmaschine in Abhängigkeit von (unter anderem) einer beispielhaft von der Verarbeitungseinheit ermittelten Strömungsgeschwindigkeit steuert bzw. regelt.

Vorzugsweise ist das Unterstützungssystem zur Durchführung eines hier vorgeschlagenen Verfahrens eingerichtet.

Nach einem weiteren Aspekt wird eine Verwendung eines Betriebsparameters einer Strömungsmaschine eines implantierten, vaskulären Unterstützungssystems zum Korrigieren einer möglichen Mehrdeutigkeit eines Messergebnisses eines Ultraschallsensors des Unterstützungssystems vorgeschlagen. Bevorzugt wird zumindest ein hier vorgeschlagenes Verfahren oder ein hier vorgeschlagenes Unterstützungssystem zum Korrigieren einer möglichen Mehrdeutigkeit eines Messergebnisses eines Ultraschallsensors verwendet.

Die im Zusammenhang mit dem Verfahren erörterten Details, Merkmale und vorteilhaften Ausgestaltungen können entsprechend auch bei dem hier vorgestellten Unterstützungssystem und/oder der Verwendung auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:

Fig. 1    ein implantierbares, vaskuläres Unterstützungssystem,
Fig. 2    das Unterstützungssystem gemäß Fig. 1, implantiert in einem Herz,
Fig. 3    ein weiteres implantierbares, vaskuläres Unterstützungssystem,
Fig. 4    das Unterstützungssystem gemäß Fig. 3, implantiert in einem Herz,
Fig. 5    eine beispielhafte Veranschaulichung einer Dopplermessung,
Fig. 6    einen Ablauf eines hier vorgestellten Verfahrens bei einem regulären Betriebsablauf,
Fig. 7    ein beispielhaftes Doppler-Frequenzspektrum,
Fig. 8    ein weiteres beispielhaftes Doppler-Frequenzspektrum, und
Fig. 9    eine funktionale Veranschaulichung einer möglichen Ausführungsform des hier vorgestellten Verfahrens.

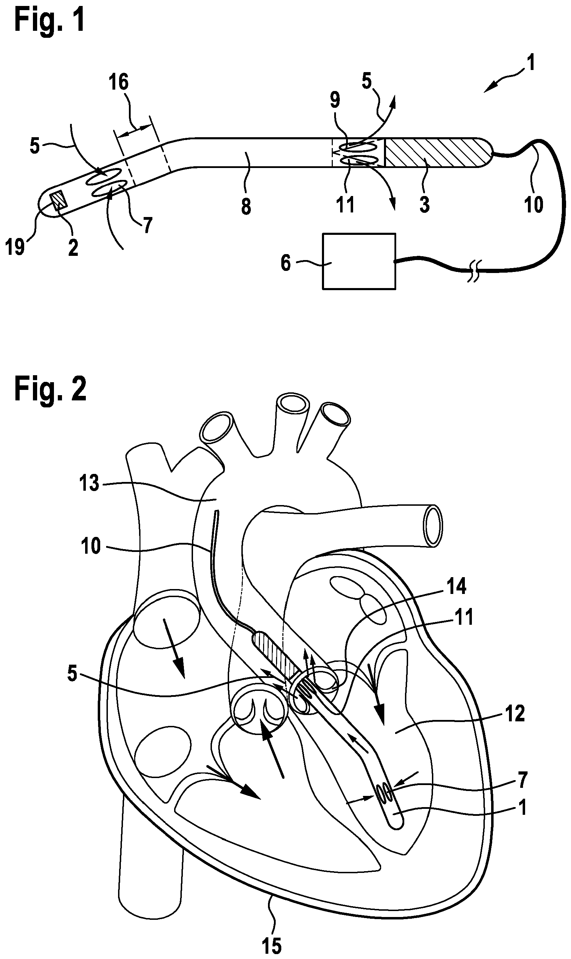

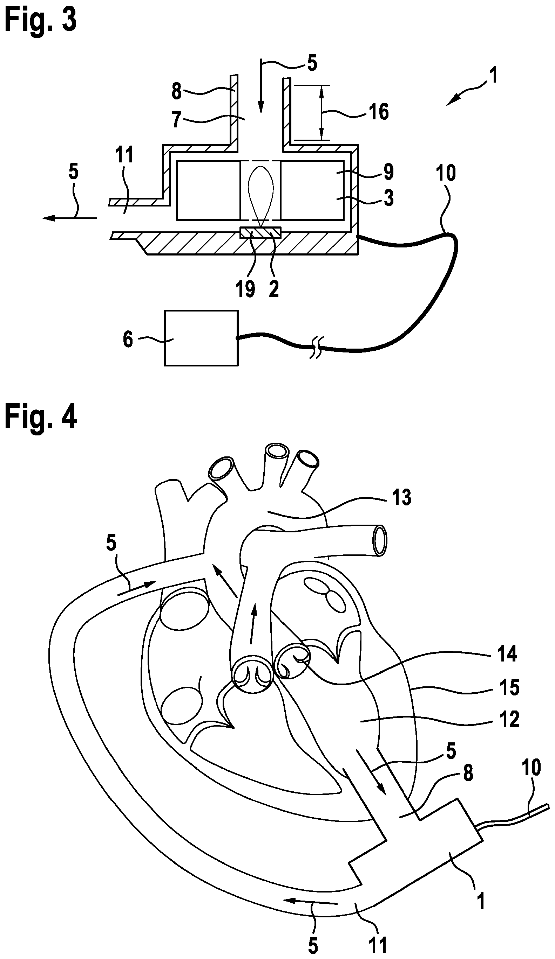

Das vaskuläre Unterstützungssystem ist bevorzugt ein ventrikuläres und/oder kardiales Unterstützungssystem bzw. ein Herzunterstützungssystem. Zwei besonders vorteilhafte Formen von Herzunterstützungssystemen sind in der Aorta platzierte Systeme nach Fig. 2 und apikal platzierte Systeme nach Fig. 4. Die jeweiligen Systeme werden im Zusammenhang mit Fig.1 (arotal) und Fig. 3 (apikal) näher erläutert.

Fig. 1 zeigt schematisch ein implantierbares, vaskuläres Unterstützungssystem 1. Dabei veranschaulicht Fig. 1 eine Ausführungsform eines aortal platzierten (vgl. Fig. 2) bzw. platzierbaren Unterstützungssystems 1. Das Unterstützungssystem 1 umfasst einen Ultraschallsensor 2, eingerichtet zur Durchführung einer gepulsten Dopplermessung, eine Strömungsmaschine 3 sowie eine Verarbeitungseinheit 6, eingerichtet zur Korrektur einer möglichen Mehrdeutigkeit eines Messergebnisses des Ultraschallsensors 2 unter Verwendung des Betriebsparameters der Strömungsmaschine 3. Der Ultraschallsensor 2 weist hier beispielhaft genau ein Ultraschall(-wandler-)element 19 auf.

Das Unterstützungssystem 1 nach Fig. 1 umfasst hier ferner beispielhaft einen distalen Teil mit Zulauföffnungen 7, durch die das Blut in das Innere des Systems angesaugt werden kann und ein Zulaufrohr 8 (das in der aortalen Ausführungsform nach Fig. 1 in der Art einer Zulaufkanüle gebildet ist). Zudem ist die Strömungsmaschine 3 beispielhaft mit einem Impeller 9 ausgestattet. Proximal am Antrieb (z. B. Elektromotor; hier nicht dargestellt) der Strömungsmaschine 3 ist hier beispielhaft ein Zuleitungskabel 10 platziert. Im Bereich des Impellers 9 befinden sich weiterhin Austrittsöffnungen 11, durch die das Blut abgegeben werden kann. Durch das Zulaufrohr 8 strömt im Betrieb ein Fluid-Volumenstrom 5, der über die Zulauföffnungen 7 in das Unterstützungssystem 1 eintritt und über die Austrittsöffnungen 11 wieder austritt. Dieser Fluid-Volumenstrom 5 kann auch als sog. Pumpen-Volumenstrom bezeichnet werden.

Fig. 2 zeigt schematisch das Unterstützungssystem 1 gemäß Fig. 1, implantiert in einem Herz 15. Die Bezugszeichen werden einheitlich verwendet, sodass auf die obigen Ausführungen Bezug genommen werden kann.

Die Zulauföffnungen 7 befinden sich im implantierten Zustand beispielsweise im Bereich des Ventrikels 12, während die Austrittsöffnungen im implantierten Zustand im Bereich der Aorta 13 liegen. Diese Ausrichtung des Unterstützungssystems 1 ist hier lediglich beispielhaft und nicht zwingend, vielmehr kann das Unterstützungssystem beispielsweise umgekehrt ausgerichtet sein. Das System wird hier weiterhin beispielhaft derart implantiert, dass es durch die Aortenklappe 14 hindurch tritt. Eine solche Anordnung kann auch als sog. Aortenklappenposition bezeichnet werden.

Fig. 3 zeigt schematisch ein weiteres implantierbares, vaskuläres Unterstützungssystem 1. Dabei veranschaulicht Fig. 3 eine Ausführungsform eines apikal platzierten (vgl. Fig. 4) bzw. platzierbaren Unterstützungssystems 1. Die Funktionsweise eines apikal implantierten Systems ist prinzipiell vergleichbar, sodass hier einheitliche Bezugzeichen für alle Komponenten verwendet werden können. Daher wird hier auf die vorstehenden Ausführungen zur Fig. 1 Bezug genommen.

Fig. 4 zeigt schematisch das Unterstützungssystem 1 gemäß Fig. 3, implantiert in einem Herz 15. Die Bezugszeichen werden einheitlich verwendet, sodass auch hier auf die obigen Ausführungen Bezug genommen werden kann.

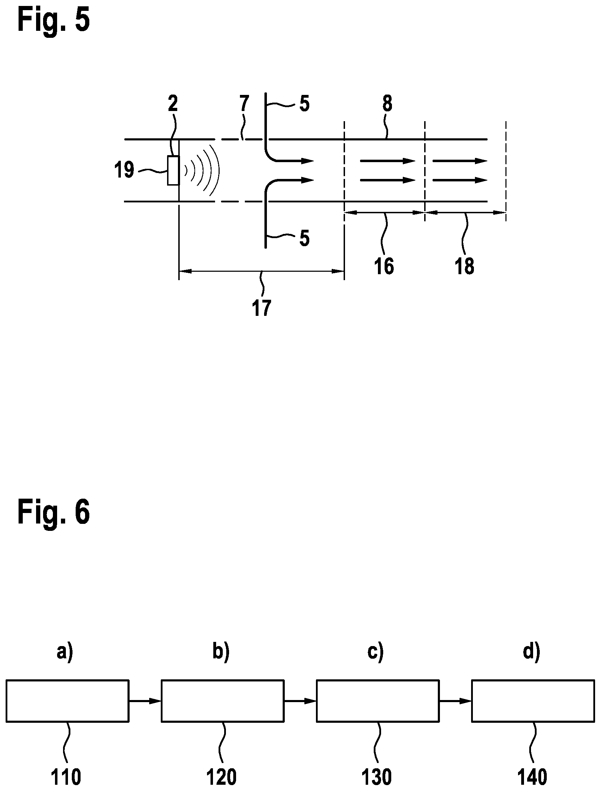

Fig. 5 zeigt schematisch eine beispielhafte Veranschaulichung einer Dopplermessung. Hierzu wird beispielhaft der Ultraschallsensor 2 des Unterstützungssystems 1 nach Fig. 1 verwendet, um eine Messung in einem Zulaufrohr 8 des Unterstützungssystems 1 nach Fig. 1 durchzuführen.

Das Messfenster, auch als Beobachtungsfenster und/oder Messbereich bezeichnet, für die Ultraschallmessung ist in den Fig. 1, 3 und 5 mit dem Bezugszeichen 16 gekennzeichnet. Die Wahl des Messfensters 16 richtet sich nach konkreter Ausgestaltung des (Herz-)Unterstützungssystems 1 und sollte grundsätzlich dort platziert werden, wo geeignete Strömungsverhältnisse herrschen. Beispielsweise zeigt Fig. 5 eine vereinfachte Schnittansicht des distalen Endes der Ausführung aus Fig. 1. Hier ist schematisch dargestellt, dass links des Messfensters 16 im Bereich 17 keine parallelen Strömungslinien vorherrschen. Da der Dopplereffekt auch vom $\cos(\alpha)$ zwischen Hauptstrahlrichtung des Ultraschallwandlers und der Hauptströmungsrichtung abhängig ist, ist es vorteilhaft, in einem Bereich paralleler Strömungslinien zu messen. Ein zu weit weg platziertes Messfenster (z. B. Bereich 18) ist zwar prinzipiell möglich, kann aber den im Folgenden erläuterten Aliasing-Effekt verschärfen und/oder für eine starke Dämpfung des Ultraschallsignals sorgen.

Der Ultraschallsensor 2 ist eingerichtet zur Durchführung einer gepulsten Dopplermessung. Hierbei wird zur Ultraschallmessung grundsätzlich das gepulste Doppler-(engl.: Pulsed Wave Doppler; kurz: PWD)-Verfahren eingesetzt. Der Ultraschallsensor 2 und die Verarbeitungseinheit 6 können daher im Folgenden auch als sog. PWD-System bezeichnet werden.

Das Messfenster 16 ist im PWD-System typischerweise elektronisch wählbar, sodass in vorteilhafter Weise durch unterschiedlich tiefe Messfenster 16 auch eine Aussage über die Strömungsverhältnisse in unterschiedlichen Bereichen der Strömungsführung getroffen werden kann.

Bei der (apikalen) Ausführungsform nach Fig. 4 strömt das Blut in entgegengesetzter Richtung auf das Ultraschallelement 19 zu. Zwischen Ultraschallelement 19 und Zulaufrohr 8 befindet sich der rotierende Impeller 9. Hier sind starke Verwirbelungen im Blutfluss zu erwarten, sodass es auch hier besonders vorteilhaft ist, das Messfenster 16 vor dem Impeller 9, etwa im Bereich des Zulaufrohrs 8 zu platzieren.

Einen großen Einfluss auf die PWD-Anwendung haben in beiden (Herz-)Unterstützungssystemvarianten, vor allem in der (aortalen) Variante nach Fig. 1, die relativ hohen Strömungsgeschwindigkeiten im Bereich des Messfensters 16 in Relation zur Entfernung des Ultraschall(-wandler-)elements 19 zum Messfenster 16.

Fig. 6 zeigt schematisch einen Ablauf eines hier vorgestellten Verfahrens bei einem regulären Betriebsablauf. Das Verfahren dient zur Bestimmung zumindest einer Strömungsgeschwindigkeit oder eines Fluid-Volumenstroms eines durch ein implantiertes, vaskuläres Unterstützungssystem 1 (vgl. Fig. 1 bis 5) strömenden Fluids. Die dargestellte Reihenfolge der Verfahrensschritte a), b), c) und d) mit den Blöcken 110, 120, 130 und 140 ist lediglich beispielhaft. In Block 110 erfolgt ein Durchführen einer gepulsten Dopplermessung mittels eines Ultraschallsensors 2 des Unterstützungssystems 1. In Block 120 erfolgt ein Auswerten eines Messergebnisses aus Schritt a), welches eine mögliche Mehrdeutigkeit aufweist. In Block 130 erfolgt ein Bereitstellen mindestens eines Betriebsparameters einer Strömungsmaschine 3 des Unterstützungssystems 1. In Block 140 erfolgt ein Ermitteln zumindest der Strömungsgeschwindigkeit oder des Fluid-Volumenstroms unter Verwendung des in Schritt b) ausgewerteten Messergebnisses. Bei dem Verfahren wird die mögliche Mehrdeutigkeit des Messergebnisses unter Verwendung des Betriebsparameters korrigiert.

Für eine exemplarische Darstellung des Verfahrens wird ein System nach Fig. 1 mit folgenden Parametern angenommen:

- Innen-Durchmesser des Zulaufrohrs: 5 mm
- Maximal zu messender Blutfluss: 9 Liter/Minute
- Resultierende maximale Flussgeschwindigkeit: 7,64 Meter/Sekunde
- Schallgeschwindigkeit in Blut: 1540 m/s
- Frequenz des Ultraschalls: 6 MHz
- Abstand vom Ultraschallwandler zum Beginn des Messfensters: 25 mm
- Ultraschall-Schwingungszyklen pro ausgesendetem Ultraschall-PWD-Impuls: 10
- Resultierende Burstlänge (10 Schwingungen bei 1540 m/s): 2,57mm

Ein Ultraschallimpuls wird am Ultraschallelement 19 ausgesendet und breitet sich in Richtung des Messfensters 16 aus. Nach dem Aussenden des Impulses schaltet das PWD-System auf Empfangsrichtung um und empfängt die kontinuierlich, beispielsweise an Streukörpern im Blut zurückgestreuten Anteile. Dabei wird die Laufzeit des Impulses vom Ultraschallelement zum Messfenster und vom Messfenster zum Ultraschallelement zurück beachtet. Im dargestellten Fall ist die gesamte relevante Ausbreitungsstrecke also 55,13 mm lang (Ultraschallelement 19 bis Beginn Messfenster 16 plus Burstlänge x 2). Frühestens, wenn das letzte Echo aus dem Bereich des Messfensters 16 eingetroffen ist, wird das PWD-System wieder auf Sendebetrieb umgeschaltet und der nächste Impuls ausgesendet. Im konkret betrachteten Fall limitiert die Pulslaufzeit die maximale Pulswiederholrate auf 27,93 kHz.

Hingegen beträgt die im dargestellten Fall auftretende maximale Doppler-Verschiebung 59,53 kHz. Bei einer komplexwertigen Auswertung (IQ-Demodulation) führt dies zu einer minimalen Pulswiederholrate von 59,53 kHz, bei der die vorliegende Dopplerverschiebung mehrdeutigkeitsfrei interpretiert werden kann. Da die Messung jedoch mit maximal 27,93 kHz (maximale Pulswiederholrate; siehe oben) erfolgt, wird hier das Nyquist-Abtasttheorem verletzt und es kommt in der Regel zu Mehrdeutigkeiten im resultierenden Doppler-Spektrum. Diese Mehrdeutigkeiten werden hier unter Verwendung eines Betriebsparameters der Strömungsmaschine des Unterstützungssystem aufgelöst, um eine eindeutige Aussage zur Hauptflussgeschwindigkeit im Beobachtungsfenster treffen zu können.

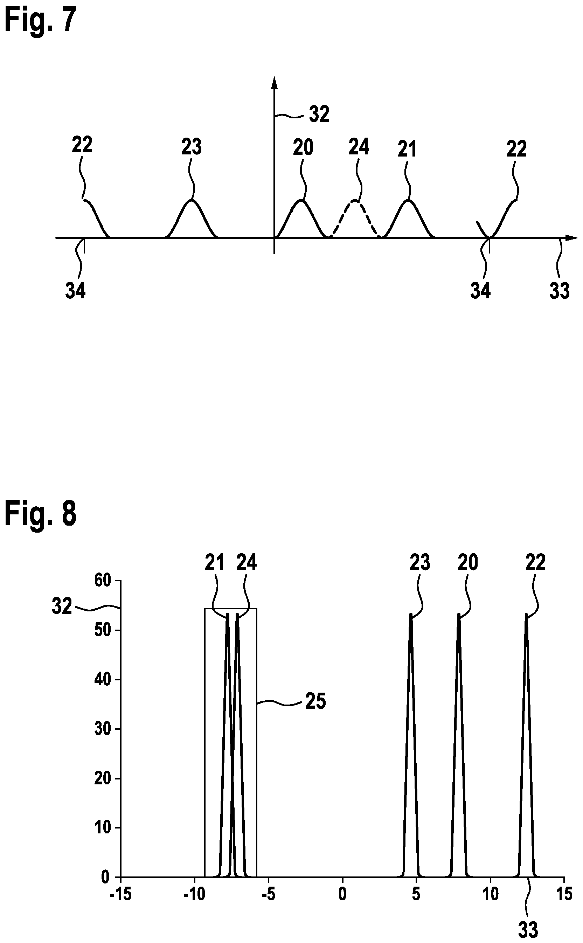

Fig. 7 zeigt schematisch ein beispielhaftes Doppler-Frequenzspektrum. Hierbei wird eine schematische Darstellung der zuvor dargelegten Zusammenhänge im Frequenzspektrum gezeigt. Eine entsprechende Darstellung der dargelegten Zusammenhänge ist zudem auch in Fig. 8 veranschaulicht.

Fig. 7 zeigt die Amplitude 32 des Doppler-Signals über der (gemittelten) Frequenz 33 bei fester Pulswiederholrate 34 (PRF). Die (feste) Pulswiederholrate 34 beträgt in dem hier betrachteten Beispiel beispielhaft 27 kHz. Gezeigt sind in Fig. 7 vereinfachte Spektren für verschiedene Strömungsgeschwindigkeiten des Fluids (hier: des Bluts). Dabei ist eine erste Strömungsgeschwindigkeit 20 kleiner als eine zweite Strömungsgeschwindigkeit 21, die wiederum kleiner ist als eine dritte Strömungsgeschwindigkeit 22, die wiederum kleiner ist als eine vierte Strömungsgeschwindigkeit 23, die wiederum kleiner ist als eine fünfte Strömungsgeschwindigkeit 24.

Es ist zu erkennen, dass es bei der dritten Strömungsgeschwindigkeit 22 bereits zu einer Verletzung des Nyquist-Theorems kommt, d. h. die Doppler-Frequenz im Bereich der Pulswiederholrate (PRF; hier beispielhaft 27 kHz) liegt. Bei weiter steigender Strömungsgeschwindigkeit des Blutes wandert das Spektrum aus dem negativen Frequenzbereich zum Koordinatenursprung. Bereits hier besteht eine Mehrdeutigkeit über die Strömungsrichtung, d. h. entweder einer schnellen Strömung auf das Ultraschallelement zu oder einer langsameren Strömung vom Ultraschallelement weg. Bei weiter steigender Strömungsgeschwindigkeit erscheint das Spektrum von der fünften Strömungsgeschwindigkeit 24 im Mehrdeutigkeitsbereich hoher oder niedriger Strömungsgeschwindigkeit.

Die hier vorgestellte Lösung erlaubt in vorteilhafter Weise eine Auflösung solcher Mehrdeutigkeiten. Hierzu kann grundsätzlich bereits eine vergleichsweise grobe Bereichsschätzung beitragen, da das Ultraschallverfahren nach wie vor hochgenau arbeitet (Auflösung auf 1-2 Nachkommastellen der Strömungsgeschwindigkeit in Meter/Sekunde bzw. des Volumenstroms in Liter/Minute), jedoch Mehrdeutigkeit über den einige Meter/Sekunde bzw. Liter/Minute großen Bereich vorliegt.

Fig. 8 zeigt schematisch ein weiteres beispielhaftes Doppler-Frequenzspektrum. Hierbei veranschaulicht Fig. 8 das Problem und die Auflösung der Mehrdeutigkeit nochmals am Beispiel mit den weiter oben verwendeten Parametern fus = 6 MHz, PRF = 27,93 kHz, $c_{Blut}$ = 1540 m/s und einer Fensterung (Fenster-Funktion) mit einem sog. Hamming-Fenster 25. Die Abbildung zeigt das Frequenzverhalten bei folgenden Flussgeschwindigkeiten:

- Erste Strömungsgeschwindigkeit 20 = -1 m/s,
- Zweite Strömungsgeschwindigkeit 21 = +1 m/s,
- Dritte Strömungsgeschwindigkeit 22 = 2 m/s,
- Vierte Strömungsgeschwindigkeit 23 = 3 m/s,
- Fünfte Strömungsgeschwindigkeit 24 = 4,5 m/s.

Eine negative Geschwindigkeit bedeutet in diesem Kontext auf das Ultraschallelement zuströmendes Blut und zeigt sich in einer Frequenzverschiebung mit positivem Vorzeichen.

Dieses Beispiel zeigt, dass die gemessenen Frequenz-Peaks bei Flussgeschwindigkeiten bei 1 m/s und 4,5 m/s sehr dicht beieinander liegen. Durch das (A priori-)Wissen der ungefähren Geschwindigkeit auf Basis des Betriebsparameters der Strömungsmaschine, kann diese Doppeldeutigkeit aufgelöst werden.

Dieses ungefähre Geschwindigkeitsintervall $v_{int}$ (plausibler Bereich der Strömungsgeschwindigkeit) kann mithilfe der nachfolgenden Formel nach einer entsprechenden Dopplerverschiebung bzw. einem Dopplerverschiebungsintervall $f_{d,int}$ aufgelöst werden.

$$f_{d,int} = \frac{2 \cdot f_0}{c_{Blut}} \cdot v_{int}$$

Im Beispiel ist das entsprechende Dopplerverschiebungsintervall 31,95 kHz bis 35,84 kHz. Um das entsprechende Frequenzintervall in den mit der verwendeten PRF darstellbaren Frequenzbereich zu verschieben, können die ermittelten, nicht darstellbaren Frequenzen mit nachfolgender Formel (für positive Flussgeschwindigkeiten) in den darstellbaren Frequenzbereich umgerechnet werden.

$$f_{d,int,PRF} = \left( f_{d,int} \bmod PRF \right) - \frac{PRF}{2}$$

Für die im Beispiel gezeigten Werte umfasst das durch den Betriebsparameter vorhersagbare Frequenzintervall also alle Frequenzen zwischen -9,95 kHz und -6,05 kHz. Alle in diesem Intervall gemessenen Frequenzen entsprechen Geschwindigkeiten im Bereich von 4,1 m/s bis 4,6 m/s.

Die exakte Geschwindigkeit (tatsächliche Strömungsgeschwindigkeit) kann durch eine Berechnung aus der Anzahl der spektralen "Wraps" mithilfe des Betriebsparameter-Intervalls (durch den Betriebsparameter vorhersagbaren Frequenzintervalls) und sukzessives Rückrechnen mit den bereits gezeigten Formeln aus der gemessenen Frequenz bestimmt werden. Als "Wrap" wird hierbei der Sprung eines Signals von der größten positiven darstellbaren Frequenz ($f_{PRF}$/2) auf die betragsmäßig größte darstellbare negative Frequenz (-$f_{PRF}$/2) bezeichnet. Die wahre Frequenz bestimmt sich nach der Formel

$$f_d = n \, f_{PRF} + f_{mess}$$

wobei der Parameter n die Anzahl der spektralen "Wraps" bezeichnet. Für geringe Strömungsgeschwindigkeiten ist $f_d$ = $f_{mess}$, bei höheren Geschwindigkeiten treten Doppeldeutigkeiten bezüglich des Werts von n auf, die gemäß der hier vorgeschlagenen Lösung durch zusätzliches Wissen (den oder die Betriebsparameter der Strömungsmaschine) aufgelöst werden können.

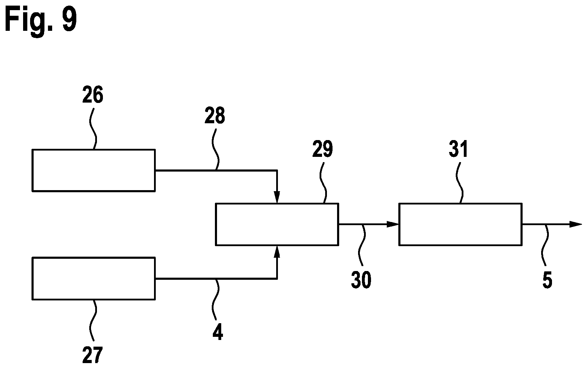

Fig. 9 zeigt schematisch eine funktionale Veranschaulichung einer möglichen Ausführungsform des hier vorgestellten Verfahrens. Das Verfahren gemäß der Darstellung nach Fig. 9 dient zur Auflösung der Mehrdeutigkeiten. Parallel oder sequentiell finden eine PWD-Volumenstrommessung 26 und eine Motorkennfeld-basierte Volumenstrommessung 27 statt. Die PWD-Volumenstrommessung 26 kann beispielsweise während Schritt a) durchgeführt werden. Die Motorkennfeld-basierte Volumenstrommessung 27 kann beispielsweise zwischen den Schritten c) und d) oder während Schritt d) durchgeführt werden. Die PWD-Volumenstrommessung 26 liefert hier ein Doppler-Spektrum 28. Dies kann beispielsweise während Schritt b) erfolgen. Die Motorkennfeld-basierte Volumenstrommessung 27 liefert einen geschätzten (Grob-)Fluid-Volumenstrom 4. Dies kann beispielsweise zwischen den Schritten c) und d) oder während Schritt d) erfolgen. Das Doppler-Spektrum 28 und der geschätzte Fluid-Volumenstrom 4 werden an eine Anti-Aliasing-Einheit 29 gesendet. Die Anti-Aliasing-Einheit 29 ermittelt aus dem geschätzten Fluid-Volumenstrom 4 den (plausiblen) Bereich, in dem die (tatsächliche) Flussgeschwindigkeit liegt und aus dem Doppler-Spektrum 28 und dem (plausiblen) Volumenstrombereich die korrigierte Flussgeschwindigkeit 30, die hier auch als (tatsächliche) Strömungsgeschwindigkeit durch das Unterstützungssystem bezeichnet wird. Die Anti-Aliasing-Einheit 29 kann beispielsweise Bestandteil der hier auch beschriebenen Verarbeitungseinheit sein. Eine Volumenstromberechnungseinheit 31 kombiniert die bekannte Querschnittsgeometrie und die bekannten, bautypspezifisch und strömungsgeschwindigkeitsabhängig ermittelten Strömungsprofile zum (tatsächlichen) Fluid-Volumenstrom 5.

Die PWD-Volumenstrommessung 26 kann dabei folgende Schritten umfassen:

- Aussenden eines Ultraschall-Impulses,
- Abwarten bis zum relevanten Echo des Messfensters,
- Empfang des Echos des Messfensters,
- Ggf. Abwarten eines weiteren Warteintervalls bis zum Abklingen entfernter Echos,
- Aussenden des nächsten Ultraschall-Impulses.

Die generierten Daten können in einem Speicher zur späteren Auswertung zwischengespeichert werden oder (beispielsweise bei paralleler Implementierung in programmierbarer Logik) direkt weiterverarbeitet werden. Während des Einlaufens des Ultraschallimpulses aus dem gewünschten Messfenster (zeitliche Begrenzung) erfolgt in der Regel eine Demodulation der empfangenen Echo-Sequenz mit der bekannten Ultraschall-Impulsfrequenz ("Heruntermischen in das Basisband"). Anschließend erfolgt in der Regel eine Transformation des gewonnenen Basisbandsignals in den Frequenzbereich (Transformation vom Zeit- in den Frequenzbereich zur Berechnung des Doppler-Spektrums).

Die Motorkennfeld-basierte Volumenstrommessung 27 (Grob-Volumenstrommmessung) kann folgende Schritte umfassen:

- Ermitteln eines Pumpen-Betriebsparameters wie Drehrate (Umdrehungen pro Minute, engl.: revolutions per minute, kurz: RPM), Leistungsaufnahme, Stromaufnahme und/oder Druckdifferenz über der Strömungsmaschine (etwa Pumpe),
- Berechnung des geschätzten Fluid-Volumenstroms über einen typspezifisch ermittelten Zusammenhang oder (beispielsweise tabellenbasierte) Interpolation aus typspezifisch ermitteltem Motorkennfeld.

Die Volumenstromberechnungseinheit 31 führt beispielsweise Folgendes aus: Multiplikation des bekannten Querschnitts im Bereich des Beobachtungsfensters 16 (Formelzeichen: A), mit der Flussgeschwindigkeit 30 (Formelzeichen: v), und einem flussgeschwindigkeitsabhängigen Strömungsprofil-Korrekturparameter (Formelzeichen f(v)). Der (tatsächliche) Fluid-Volumenstrom (Formelzeichen $Q_p$) kann sich hierbei nach folgender Formel ergeben:

$$Q_p = f(v) \times v \times A$$

Die Anti-Aliasing-Einheit 29 und die Volumenstromberechnungseinheit 31 können auch zu einer Einheit kombiniert werden. Zudem kann beispielsweise das Doppler-Spektrum direkt auf den Volumenstrom $Q_p$ abgebildet werden.
Die hier vorgestellte Lösung ermöglich insbesondere einen oder mehrere der nachfolgenden Vorteile:

- Hochgenaue Berechnung des Pumpenvolumenstroms mittels Doppler-Ultraschall-Sensor.
- Kombination einer hochgenauen Doppler-Ultraschallmessung und einer groben Abschätzung auf Basis von Motor-Betriebsparametern (z. B. ein oder mehrere von Drehzahl, Strom, Leistung, aufgebauter Druck) ermöglicht den Betrieb der Ultraschall-Messung unter Verletzung des Nyquist-Theorems (Notwendigkeit aus geometrischen Gegebenheiten) und einer anschließenden Auflösung entstandener Mehrdeutigkeiten mit Hilfe der groben Abschätzung.

**Patentansprüche**

1.  Verfahren zur Bestimmung zumindest einer Strömungsgeschwindigkeit oder eines Fluid-Volumenstroms (5) eines durch ein implantiertes, vaskuläres Unterstützungssystem (1) strömenden Fluids, umfassend folgende Schritte:

    a) Durchführen einer gepulsten Dopplermessung mittels eines Ultraschallsensors (2) des Unterstützungssystems (1), wobei eine maximale Pulswiederholrate der gepulsten Dopplermessung kleiner ist als das Zweifache einer maximal auftretenden Doppler-Verschiebung,

b) Auswerten eines Messergebnisses aus Schritt a), welches eine mögliche Mehrdeutigkeit aufweist,

c) Bereitstellen mindestens eines Betriebsparameters einer Strömungsmaschine (3) des Unterstützungssystems (1),

d) Ermitteln zumindest der Strömungsgeschwindigkeit oder des Fluid-Volumenstroms (5) unter Verwendung des in Schritt b) ausgewerteten Messergebnisses,

wobei die mögliche Mehrdeutigkeit des Messergebnisses unter Verwendung des Betriebsparameters korrigiert wird.

2. Verfahren nach Anspruch 1, wobei in Schritt a) ein neuer Ultraschall-Impuls erst ausgesendet wird, wenn ein Echo eines unmittelbar zuvor ausgesendeten Ultraschall-Impulses abgeklungen ist.

3. Verfahren nach einem der vorhergehenden Ansprüche, wobei der Betriebsparameter zumindest eine Drehzahl, ein Strom, eine Leistung oder ein Druck ist.

4. Verfahren nach einem der vorhergehenden Ansprüche, wobei mit dem Betriebsparameter zumindest eine geschätzte Strömungsgeschwindigkeit oder ein geschätzter Fluid-Volumenstrom (4) ermittelt wird.

5. Verfahren nach einem der vorhergehenden Ansprüche, wobei mit dem Betriebsparameter ein plausibler Bereich bestimmt wird, in welchem plausible Messergebnisse liegen können.

6. Verfahren nach einem der vorhergehenden Ansprüche, wobei unter Verwendung der Strömungsgeschwindigkeit ein Fluid-Volumenstrom (5) durch das Unterstützungssystem (1) ermittelt wird.

7. Implantierbares, vaskuläres Unterstützungssystem (1), umfassend:

- einen Ultraschallsensor (2), eingerichtet zur Durchführung einer gepulsten Dopplermessung, wobei eine maximale Pulswiederholrate der gepulsten Dopplermessung kleiner ist als das Zweifache einer maximal auftretenden Doppler-Verschiebung,

- eine Strömungsmaschine (3),

- eine Verarbeitungseinheit (6), eingerichtet zur Korrektur einer möglichen Mehrdeutigkeit eines Messergebnisses des Ultraschallsensors (2) unter Verwendung des Betriebsparameters der Strömungsmaschine (3).

8. Unterstützungssystem nach Anspruch 7, eingerichtet zur Durchführung eines Verfahrens nach einem der Ansprüche 1 bis 6.

9. Verwendung eines Betriebsparameters einer Strömungsmaschine (3) eines implantierten, vaskulären Unterstützungssystems (1) nach Anspruch 8 zum Korrigieren einer möglichen Mehrdeutigkeit eines Messergebnisses eines Ultraschallsensors (2) des Unterstützungssystems (1).

**Claims**

1. Method for determining at least one flow rate or a fluid volume flow (5) of a fluid flowing through an implanted, vascular support system (1), comprising the following steps:

a) carrying out a pulsed Doppler measurement by means of an ultrasound sensor (2) of the support system (1), wherein a maximum pulse repetition rate of the pulsed Doppler measurement is less than twice a maximum occurring Doppler shift,

b) evaluating a measurement result from step a) which has a possible ambiguity,

c) providing at least one operating parameter of a flow machine (3) of the support system (1),

d) determining at least the flow rate or the fluid volume flow (5) using the measurement result evaluated in step b),

wherein the possible ambiguity of the measurement result is corrected using the operating parameter.

2. Method according to claim 1, wherein, in step a), a new ultrasound pulse is only emitted when an echo of an immediately preceding ultrasound pulse has faded away.

3. Method according to either of the preceding claims, wherein the operating parameter is at least one rotational speed, current, power, or pressure.

4. Method according to any of the preceding claims, wherein at least one estimated flow rate or estimated fluid volume flow (4) is determined using the operating parameter.

5. Method according to any of the preceding claims, wherein a plausible region in which plausible measurement results can be is determined using the operating parameter.

6. Method according to any of the preceding claims, wherein a fluid volume flow (5) is determined by the support system (1) using the flow rate.

7. Implantable, vascular support system (1) comprising:

   - an ultrasound sensor (2) which is configured to carry out a pulsed Doppler measurement, wherein a maximum pulse repetition rate of the pulsed Doppler measurement is less than twice a maximum occurring Doppler shift,
   - a flow machine (3),
   - a processing unit (6) which is configured to correct a possible ambiguity of a measurement result of the ultrasound sensor (2) using the operating parameter of the flow machine (3).

8. Support system according to claim 7, which is configured to carry out a method according to any of claims 1 to 6.

9. Use of an operating parameter of a flow machine (3) of an implanted, vascular support system (1) according to claim 8 for correcting a possible ambiguity of a measurement result of an ultrasound sensor (2) of the support system (1).

**Revendications**

1. Procédé permettant de déterminer au moins une vitesse d'écoulement ou un débit volumétrique de fluide (5) d'un fluide s'écoulant à travers un système d'assistance (1) vasculaire implanté, comprenant les étapes suivantes :

   a) réalisation d'une mesure Doppler pulsée au moyen d'un capteur à ultrasons (2) du système d'assistance (1), dans lequel un taux de répétition d'impulsions maximal de la mesure Doppler pulsée est inférieur au double d'un décalage Doppler maximal se produisant,
   b) évaluation d'un résultat de mesure de l'étape a) qui présente une éventuelle ambiguïté,
   c) fourniture d'au moins un paramètre de fonctionnement d'une turbomachine (3) du système d'assistance (1),
   d) définition d'au moins la vitesse d'écoulement ou le débit volumétrique de fluide (5) à l'aide du résultat de mesure évalué à l'étape b),

   dans lequel l'éventuelle ambiguïté du résultat de mesure est corrigée à l'aide du paramètre de fonctionnement.

2. Procédé selon la revendication 1, dans lequel, à l'étape a), une nouvelle impulsion ultrasonore n'est émise que lorsqu'un écho d'une impulsion ultrasonore émise immédiatement avant a disparu.

3. Procédé selon l'une des revendications précédentes, dans lequel le paramètre de fonctionnement est au moins une vitesse de rotation, un débit, une puissance ou une pression.

4. Procédé selon l'une des revendications précédentes, dans lequel, au moyen du paramètre de fonctionnement, au moins une vitesse d'écoulement estimée ou un débit volumétrique de fluide (4) estimé est défini.

5. Procédé selon l'une des revendications précédentes, dans lequel, au moyen du paramètre de fonctionnement, une plage plausible, dans laquelle des résultats de mesure plausibles peuvent se trouver, est déterminée.

6. Procédé selon l'une des revendications précédentes, dans lequel un débit volumétrique de fluide (5) est défini par le système d'assistance (1) à l'aide de la vitesse d'écoulement.

7. Système d'assistance (1) vasculaire implantable, comprenant :

- un capteur à ultrasons (2), configuré pour la réalisation d'une mesure Doppler pulsée, dans lequel un taux de répétition d'impulsions maximal de la mesure Doppler pulsée est inférieur au double d'un décalage Doppler maximal se produisant,
- une turbomachine (3),
- une unité de traitement (6), configurée pour la correction d'une éventuelle ambiguïté d'un résultat de mesure du capteur à ultrasons (2) à l'aide du paramètre de fonctionnement de la turbomachine (3).

8. Système d'assistance selon la revendication 7, configuré pour la réalisation d'un procédé selon l'une des revendications 1 à 6.

9. Utilisation d'un paramètre de fonctionnement d'une turbomachine (3) d'un système d'assistance (1) vasculaire implanté selon la revendication 8 pour la correction d'une éventuelle ambiguïté d'un résultat de mesure d'un capteur à ultrasons (2) du système d'assistance (1).

# Fig. 1

# Fig. 2

**Fig. 3**

**Fig. 4**

# Fig. 5

# Fig. 6

# Fig. 7

# Fig. 8

# Fig. 9

**IN DER BESCHREIBUNG AUFGEFÜHRTE DOKUMENTE**

**In der Beschreibung aufgeführte Patentdokumente**

- DE 10060275 A1